# EUROPÄISCHE PATENTSCHRIFT

(11) **EP 0 473 987 B1**
(45) Veröffentlichungstag und Bekanntmachung des Hinweises auf die Patenterteilung: **02.11.1995**
(21) Anmeldenummer: 91113625.7
(22) Anmeldetag: 14.08.1991
(51) Int. Cl.: A61B 17/36

(54) **Verfahren und Vorrichtung zur optoelektrischen Erkennung und Unterscheidung von an einen Laser angeschlossenen medizinischen Einwegapplikatoren**
Method and apparatus for optoelectrical recognition of disposable medical applicators connected to a laser
Méthode et dispositif pour la détection et la distinction opto-électriques d'applicateurs médicaux jetables raccordés à un laser

(30) Priorität: 21.08.1990 DE 4026452
(43) Veröffentlichungstag der Anmeldung: 11.03.1992
(73) Patentinhaber: Schott Glaswerke, 55122 Mainz (DE); Carl-Zeiss-Stiftung trading as SCHOTT GLASWERKE, 55122 Mainz (DE)
(72) Erfinder: Kobayashi, Noboru, Dipl.-Ing., W-6500 Mainz (DE)
(74) Vertreter: Fuchs Mehler Weiss

(56) Entgegenhaltungen:
- EP-A- 0 300 317
- EP-A- 0 342 772
- EP-A- 0 408 160
- DE-A- 2 515 656
- GB-A- 2 210 706
- US-A- 4 907 588

## Beschreibung

Die Erfindung betrifft ein Verfahren zur Erkennung und Unterscheidung von über eine Steckverbindung an einen Laser anschließbaren medizinischen Einwegapplikatoren.

Im medizinischen Bereich werden Applikatoren unter anderem dazu verwendet, Laserlicht über Lichtleitfasern vom Laserkopf zum Einsatzpunkt zu transportieren. Entsprechend den unterschiedlichen Aufgaben, die das Laserlicht am distalen Austrittsende des Lichtleiters jeweils zu erfüllen hat, sind die zum Einsatz kommenden Applikatoren unterschiedlich dimensioniert und in ihrer Auslegung auf den spezifischen Einsatz optimiert. Demzufolge gibt es eine Vielzahl von unterschiedlichen Applikatortypen und Ausführungsformen, die noch dazu in unterschiedliche Laser-Arten eingekoppelt werden müssen.

Die für die Einkopplung der Applikatoren in den Laser erforderliche Steckverbindung stellt bisher eine Schwachstelle des Systems dar. Zum einen müssen an die Steckverbindung erhebliche Anforderungen gestellt werden, z.B. einfaches Verbinden und Lösen, einfache Montage (auch am Einsatzort), mechanischer Schutz, einfaches Säubern und Instandsetzen, reproduzierbare Positioniergenauigkeit auch nach wiederholtem Stecken, und anderes mehr, zum anderen sollen die Applikatoren gerade bei medizinischer Anwendung in möglichst sterilem Zusand zum Einsatz kommen.

Die bisher in der Medizin verwendeten Applikator-Typen benutzen als Einkoppelstecker Linsenstecker mit Metallfassung, um den beim Sterilisieren (überwiegend im Autoklaven) auftretenden Belastungen, besonders im Hinblick auf Temperaturresistenz, standhalten zu können. Zur Wiederverwendbarkeit der teuren Linsenstecker muß jedoch spätestens nach zwei- bis dreimaligem Sterilisieren eine Neueinstellung der Optik vorgenommen werden, die umständlich und arbeitsaufwendig von Hand vorgenommen werden muß. Andererseits sind aber auch seit geraumer Zeit linsenfreie, genormte Standardstecker mit im Vergleich zu den Linsensteckern relativ kleinen Abmessungen im Handel, die billig hergestellt werden können, die dann aber im medizinischen Bereich wegen der beim Sterilisieren auftretenden hohen Anforderungen an die Temperaturbelastbarkeit nicht wiederverwendbar wären. Wegen ihrer geringen Herstellungskosten könnten sie integriert mit den Applikatoren als Einweg-Artikel eingesetzt werden. Dem steht aber entgegen, daß wegen der kleinen Dimensionierung und der Uniformität der Stecker das Risiko einer Verwechslung der für den jeweils spezifischen medizinischen Einsatz geeigneten Applikatoren mit dafür ungeeigneten Applikatoren sehr groß ist. Es ist daher wünschenswert, ein Verfahren zur Verfügung zu haben, mit dem die unterschiedlichen Applikatortypen vor ihrem eigentlichen Einsatz schnell und unkompliziert erkannt und voneinander unterschieden werden können.

Aus der US-PS 4,907,588 ist ein Einkoppelsystem bekannt, in welchem über optische Kennung zwischen Laserkopf und Einkoppelstecker mit Hilfe einer nachgeschalteten Kontrolleinheit die Intensität und Wellenlänge des Strahls eines Mehrbereichslasers auf die Optik des Applikatorsteckers eingestellt werden kann. Der Laserstrahl läßt sich dabei aber nur bei schon eingeschaltetem Gerät nach der Optik des Steckers ausrichten. Aussagen über die Eignung oder Nichteignung des verwendeten Applikators können damit nicht erhalten werden, zumal Stecker und Applikator keine integrierte Einheit bilden.

Aus der DE-A-2 515 656 wird eine optische Abtastvorrichtung zur Identifizierung von Körpern beschrieben, wobei die Reihenfolge von auf dem Körper angebrachten Farbmarken mittels sequentiell gezündeter Blitzlichtröhren abgetastet und bestimmt wird. Das beim Anblitzen von den Farbmarken reflektierte Licht wird von Photozellen aufgenommen, die so angeordnet sind, daß das reflektierte Licht aller Farbmarken gleichmäßig auf alle Photozellen trifft. Die einzelnen Photozellen sind verschieden farbempfindlich, indem ihre Farbempfindlichkeit den Farben der entsprechenden Farbmarken entspricht. Die sequentiellen Steuerspannungen für die Blitzlichtröhren und die Spannungen der einzelnen Photozellen werden einer Auswerteschaltung zugeführt, wo eine zuvor eingegebene Reihenfolge von Farbmarken mit der Reihenfolge der Farbmarken auf dem Körper verglichen wird, um so eine Übereinstimmung oder Nichtübereinstimmung festzustellen.

Eine solche Anordnung ist jedoch wegen des relativ großen Platzbedarfs der einer jeden Farbmarkierung zugeordneten Blitzröhren für die Erkennung von mehreren, auf dem Stecker eines Applikators angebrachter Farbmarken nicht geeignet.

Es ist daher Aufgabe der Erfindung, ein Verfahren zur Erkennung und Unterscheidung von unterschiedlichen medizinischen Applikatoren zur Verfügung zu stellen, wobei im Handel befindliche, genormte linsenfreie Standardstecker zur Anwendung kommen. Eine weitere Aufgabe der Erfindung soll in der Möglichkeit bestehen, geeignete Applikator-Typen für einen zuvor definierten medizinischen Einsatz von dafür ungeeigneten unterscheiden zu können, noch bevor der Laser eingeschaltet wird. Schließlich soll das Verfahren so konzipiert sein, daß die niedrigen Fertigungskosten des Applikators mit integriertem Stecker seine Verwendung als Einweg-Artikel möglich machen.

Diese Aufgabe wurde verfahrensmäßig durch die kennzeichnenden Merkmale des Anspruchs 1 gelöst.

Im Laserkopf, im Bereich der Aufnahmebuchse für den Stecker, werden Strahlquellen und Detektoren so angebracht, daß eine oder mehrere Strahlquellen bei eingekoppeltem Stecker an dessen farbmarkierten Bereich einen Strahl senden und die vom markierten Bereich des Einkoppelsteckers reflektierten Strahlen von den jeweils den Strahlquellen zugeordneten Fotodetektoren empfangen werden. Das von den Strahlquellen ausgesandte Licht kann kontinuierlich oder gepulst sein.

Der farbmarkierte Bereich kann aus mehreren unterschiedlich gefärbten Farbzonen, vorzugsweise als Farbringe am Stecker ausgebildet, bestehen. In diesem Falle sind jeder einzelnen Farbzone zwei oder vier örtlich auf die jeweilige Farbzone fixierte Strahlquellen mit jeweils dazugehörendem Detektor zugeordnet. Das von einer spezifischen Strahlquelle abgegebene Licht ist monochromatisch. Der einer Strahlquelle jeweils zugeordnete Fotodetektor kann mit oder ohne Farbfilter ausgebildet sein.

Werden pro Farbzone zwei Strahlquellen verwendet, dann geben die Strahlquellen monochromatisches Licht im Bereich von 400-780 nm mit jeweils solchen Wellenlängen ab, deren Farben zueinander komplementär sind. Das von jeweils einem Detektor an die Auswertungseinheit weitergegebene Signal wir dabei digital verarbeitet, d.h. entweder mit dem logischen Stand 0 oder 1 korreliert. Eine Farbzone wird also durch zwei Binärsignale charakterisiert.

Eine Steigerung der mittels einer Farbzone erreichbaren Information kann erhalten werden, wenn pro Farbzone vier Strahlquellen verwendet werden, von denen jeweils zwei mit Wellenlängen abstrahlen, deren jeweilige Farbkorrelationen komplementäre Farben ergeben. So können die Strahlquellen des einen Paares Rotlicht bzw. Grünlicht aussenden, die Strahlquellen des anderen Paares Blau- bzw. Gelblicht. Eine Farbzone wird dann über vier Binärsignale charakterisiert.

Die Erfindung wird im folgenden anhand eines Ausführungsbeispiels näher erläutert.

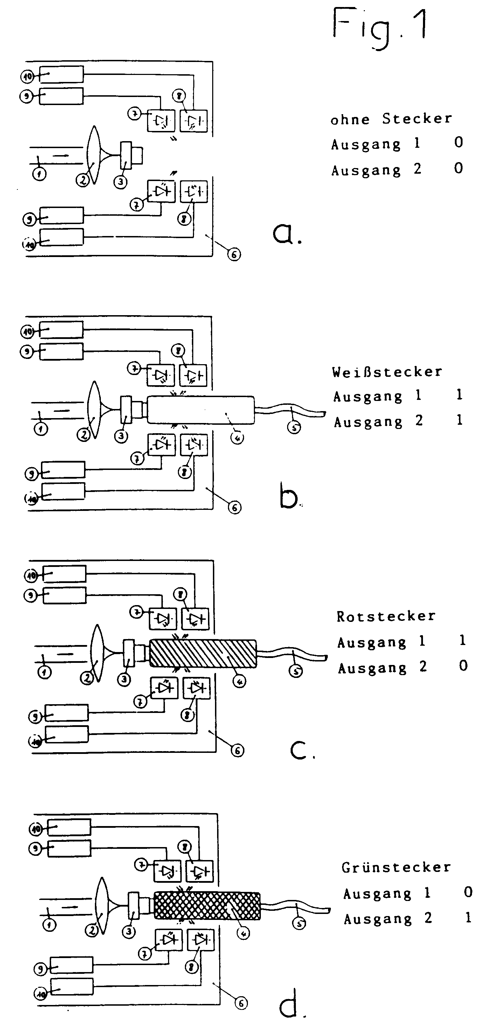

- Fig. 1: zeigt ein Laserstecker-Erkennungssytem mit digitaler Signal-Verarbeitung, in dem pro Farbzone zwei Strahlquellen mit komplementärer Farbabstrahlung eingesetzt sind.

In Fig. 1 sind zur Erkennung einer Farbzone zwei jeweils mit komplementärer Farbe abstrahlende Strahlquellen vorgesehen. Die Bearbeitung der aus den entsprechenden Fotodetektoren eintreffenden Signale erfolgt so, daß die in der Elektronik 10 eintreffenden elektrischen Signale bei Registrierung einen Schwellenwert überschreiten müssen; ansonsten, wenn die Energiegröße des elektrischen Signals diesen Schwellenwert nicht überschreitet, wird es nicht registriert. Dadurch wird eine digitale Erfassung des reflektierten Lichtes ermöglicht.

In Fig. 1 wird beispielhaft jeweils vom oberen Sendesystem 7, 9 rotes Licht abgestrahlt und vom unteren Sendesystem 7, 9 grünes Licht. Dem roten Licht wird auf der Empfangsseite 8, 10 der Ausgang 1 zugeordnet, dem grünen Licht der Ausgang 2.

Ist, wie in Fig. 1a dargestellt, kein Stecker eingekoppelt, so wird weder rotes noch grünes Licht reflektiert. Die entsprechenden Ausgangssignale lassen sich durch den dualen Ausdruck 0 0 wiedergeben.

Bei eingekoppeltem Stecker mit weißer Farbzone (Fig. 1b) wird sowohl Rot- als auch Grünlicht reflektiert. Als Ausgangssignale erhält man den logischen Stand 1 1.

Im Falle von Fig. 1c wird ein Stecker mit einer roten Farbzone eingekoppelt. Hier wird nur rotes Licht mit genügender Intensität reflektiert, um als Ausgangssignal den logischen Stand 1 zu ergeben. Die Reflexion beim grünen Licht ist zu gering, so daß sich dann ausgangsseitig die duale Kombination 1 0 ergibt.

Beim Einkoppeln eines Steckers mit einer grünen Farbzone (Fig. 1d) drehen sich die Verhältnisse gerade um, im Ausgang erhält man den logischen Stand 0 1.

Beispiel der Fig. 1, mit jeweils zwei einer Farbzone zugeordneten Strahlquellen, ergeben sich somit pro Farbzone vier verschiedene Variationen, nämlich die logischen Stände 0 0, 1 1, 1 0, 0 1.

Der Farbbereich des Applikators soll nun als Kodierung für die technischen Eigenschaften der mit dem Stecker verbundenen Applikatoren dienen. Die Möglichkeiten zur Erhöhung der Variationsbreite können dann erheblich gesteigert werden, wenn man einen Farbbereich in mehrere Farbzonen unterteilt, und, wie oben beschrieben, jeder einzelnen Farbzone jeweils ein Sende- und Empfangssystem, bestehend aus zwei oder vier Strahlquellen mit dazugehörigen Fotodetektoren, zuordnet.

Die charakteristischen Ausgangssignale für den Zustand "nichtangekoppelter Stecker" können dazu benutzt werden, mit Hilfe nachgeschalteter Elektronik den Betrieb des Lasers automatisch zu blockieren (Interloc). Im Bedarfsfall kann diese Anzeige mit optischen und/oder akustischen Signalen begleitet werden. Über die Farbkodierung der technischen Eigenschaften kann das Erkennungssystem auch dazu dienen, unter Verwendung geeigneter Software darüber zu befinden, ob der eingekoppelte Applikator für einen bestimmten gewünschten Einsatz geeignet ist oder nicht. Die Anzeige dazu könnte z.B. über ein Display erfolgen.

Da sämtliche technisch aufwendigeren Einrichtungen des Erkennungssystems, wie z.B. die den einzelnen Farbzonen zugeordneten Strahlquellen mit Fotodetektoren, im Laserkopf montiert sind, ist die Herstellung der mit dem Stecker integrierten Applikatoren selbst relativ einfach und wegen der Möglichkeit, steckerseitig auch ohne aufwendige Linsensysteme auszukommen, kostengünstig. Darüber hinaus lassen sich solche Applikatoren leicht sterilisieren. Die in der Medizin üblichen Autoklaventemperaturen beeinträchtigen, insbesondere bei einmaliger Verwendung, die Funktion des Applikators nicht. Die Stecker selbst können in allen Ausführungsformen der handelsüblichen Standardsysteme (z.B. F-SMA, FC und DIN), u.a. auch in der Ausführung als Mehrsystem-Stecker, zur Anwendung kommen. Zwischen dem Applikatorstecker und dem Laserkopf selbst ist zur Applikatorerkennung kein mechanischer und/oder elektrischer Kontakt notwendig. Daher unterliegt das einkoppelseitige Ende des Laserkopfes fast keinem Verschleiß. An die mechanische Paßgenauigkeit und die elektrische Anpassung der Applikatorstecker bei einmaliger Anwendung müssen keine allzu großen Erwartungen gestellt werden. Dies alles begünstigt ihre Verwendung als Einweg-Applikatoren, die aus hygienischen Gründen dringend erforderlich sind.

## Patentansprüche

1. Verfahren zur Erkennung und Unterscheidung von über eine Steckverbindung an einen Laser anschließbaren medizinischen Einwegapplikatoren mit integriertem Stecker, wobei vor Beaufschlagung des Applikators mit Laserlicht über im Laserkopf angeordnete Strahlquellen und ihnen zugeordneten Detektoren farbmarkierte Kodierungszonen, welche die technischen Eigenschaften und die jeweiligen medizinischen Anwendungsmöglichkeiten des Applikators codieren, auf dem Applikatorstecker auf optischem Wege abgetastet und die von den Photodetektoren gelieferten Signale an eine elektronische Auswertungseinheit weitergeleitet und auf einem Display die Eignung oder Nichteignung des Appliaktors für die zuvor der Auswertungseinheit eingegebene Anwendung angezeigt werden,
dadurch gekennzeichnet, daß jeder Farbzone in dem am Stecker des Einwegapplikators angebrachten Farbmarkierungsbereich ein oder zwei Paare monochromatischer Strahlquellen mit zugehörigen Detektoren zugeordnet werden, wobei von diesen Strahlquellen im sichtbaren Bereich des Spektrums jeweils ein Strahl mit solcher Wellenlänge auf die zugeordnete Farbzone gerichtet wird, daß sich die Strahlen der einzelnen Strahlquellen in ihren Spektralfarben voneinander unterscheiden und die entsprechenden Spektralfarben eines jeweiligen Paares komplementär zueinander sind.

2. Verfahren nach Anspruch 1, dadurch gekennzeichnet, daß von den Photodetektoren nur Licht der Wellenlänge registriert wird, die dem monochromatischen Licht der jeweils zugeordneten Strahlquelle entspricht.

3. Verfahren nach Anspruch 1 oder 2, dadurch gekennzeichnet, daß die von den jeweiligen Detektoren an die Auswertungseinheit weitergegebenen Signale digital verarbeitet werden.

4. Einwegapplikator zur Durchführung des Verfahrens nach einem der Ansprüche 1-3, dadurch gekennzeichnet, daß er einen an seiner Außenseite mit Farbmarkierungen versehenen linsenlosen Stecker umfaßt, wobei über die Farbmarkierungen die technischen Eigenschaften und die jeweiligen medizinischen Anwendungsmöglichkeiten des Applikators kodiert sind.

5. Einwegapplikator nach Anspruch 4, dadurch gekennzeichnet, daß die Farbmarkierungen aus mehreren unterschiedlich gefärbten Farbzonen bestehen.

6. Einwegapplikator nach Anspruch 5, dadurch gekennzeichnet, daß die Farbzonen in Form von Ringen ausgebildet sind.

7. Einwegapplikator nach einem der Ansprüche 4-6, dadurch gekennzeichnet, daß er mindestens eine Behandlung im Autoklaven übersteht.

## Claims

1. A process for recognizing and differentiating medical disposable applicators which can be attached to a laser by way of a plug connection and which have an integrated plug, where, before the applicator is acted upon by laser light, colour-labelled coding zones which encode the technical properties and the respective medical application Possibilities of the applicator being scanned by way of beam sources arranged in the laser head and detectors associated therewith at the applicator plug by optical means, and where the signals supplied by the photodetectors are passed on to an electronic evaluation unit and the suitability or unsuitability of the applicator for the application previously input to the evaluation unit is displayed on a display, characterized in that there are associated with each colour zone in the colour-labelling region mounted on the plug of the disposable applicator one or two pairs of monochromatic beam sources with associated detectors, where these beam sources direct at the associated colour zone, in the visible range of the spectrum, a respective beam having a wavelength such that the beams of the individual beam sources differ from one another in their spectral colours and the corresponding spectral colours of a respective pair are complementary to one another.

2. A process according to Claim 1, characterized in that the photodetectors only register light of the wavelength corresponding to the monochromatic light of the respectively associated beam source.

3. A process according to Claim 1 or 2, characterized in that the signals passed on to the evaluation unit by the respective detectors are processed digitally.

4. A disposable applicator for carrying out the process according to one of Claims 1 - 3, characterized in that it comprises a plug which is provided on its outside with colour labelling and has no lens, where the technical properties and the respective medical application possibilities of the applicator are encoded by way of the colour labelling.

5. A disposable applicator according to Claim 4, characterized in that the colour labelling comprises a plurality of differently coloured colour zones.

6. A disposable applicator according to Claim 5, characterized in that the colour zones are constructed in the form of rings.

7. A disposable applicator according to one of Claims 4 - 6, characterized in that it withstands at least one treatment in an autoclave.

## Revendications

1. Procédé de reconnaissance et de différenciation d'applicateurs médicaux jetables à connecteur intégré, susceptibles d'être raccordés par une connexion à un laser, des plages de codage munies de marques de couleur et codant les caractéristiques techniques et les utilisations médicales de chaque applicateur étant, avant l'alimentation de l'applicateur en lumière laser, explorées par voie optique sur le connecteur de l'applicateur, en passant par des sources de rayonnement disposées dans la tête laser et des détecteurs associés à ces sources, les signaux fournis par les photodétecteurs étant transmis à une unité électronique d'évaluation et la compatibilité ou incompatibilité de l'applicateur avec l'utilisation introduite auparavant dans l'unité d'évaluation étant visualisée sur un élément d'affichage, **caractérisé** par le fait qu'à chaque zone de couleur dans la plage de marques de couleur disposée sur le connecteur de l'applicateur jetable sont associées une ou deux paires de sources de rayonnement monochromatiques avec détecteurs associés, ces sources de rayonnement dirigeant, dans la plage visible du spectre, chacune un rayon d'une longueur d'onde telle sur la zone de couleur associée que les rayons des différentes sources de rayonnement diffèrent les uns des autres dans leurs couleurs spectrales et les couleurs spectrales correspondantes de chaque paire soient complémentaires l'une de l'autre.

2. Procédé suivant la revendication 1, **caractérisé** par le fait que les photodétecteurs n'enregistrent que la lumière ayant la longueur d'onde correspondant à la lumière monochromatique de la source de rayonnement associée.

3. Procédé suivant la revendication 1 ou 2, **caractérisé** par le fait que les signaux transmis par les détecteurs à l'unité d'évaluation sont traités par voie numérique.

4. Applicateur jetable pour la mise en oeuvre du procédé suivant l'une des revendications 1 à 3, **caractérisé** par le fait qu'il comprend un connecteur sans lentille muni de marques de couleur sur son côté extérieur, les marques de couleur constituant un codage des caractéristiques techniques et des utilisations médicales de l'applicateur.

5. Applicateur jetable suivant la revendication 4, **caractérisé** par le fait que les marques de couleur se composent de plusieurs zones de couleurs différentes.

6. Applicateur jetable suivant la revendication 5, **caractérisé** par le fait que les zones de couleurs sont réalisées sous la forme d'anneaux.

7. Applicateur jetable suivant l'une des revendications 4 à 6, **caractérisé** par le fait qu'il supporte au moins un traitement en autoclave.
